(19) Europäisches Patentamt
European Patent Office
Office européen des brevets

(11) Publication number: **0 046 054**
**A1**

(12) **EUROPEAN PATENT APPLICATION**

(21) Application number: 81303574.8

(22) Date of filing: 05.08.81

(51) Int. Cl.[3]: C 25 B 3/02
C 25 B 11/06, C 07 C 53/08
C 07 C 55/02, C 07 C 55/22
C 07 C 49/10

(30) Priority: 08.08.80 US 176413

(43) Date of publication of application: 17.02.82 Bulletin 82/7

(84) Designated Contracting States: BE CH DE FR GB IT LI NL SE

(71) Applicant: Exxon Research and Engineering Company
P.O.Box 390 200 Park Avenue
Florham Park New Jersey 07932(US)

(72) Inventor: Horowitz, Harold Saul
7 Timber Road
Edison New Jersey(US)

(72) Inventor: Horowitz, Hugh Harris
215 Keats Avenue
Elizabeth New Jersey(US)

(72) Inventor: Longo, John Michael
53 Woodbine Circle
New Providence New Jersey(US)

(74) Representative: Field, Roger Norton et al,
5 Hanover Square
London W1R OHQ(GB)

(54) **A method of electrocatalytic oxidation of organic compounds.**

(57) A method for electrocatalytically reacting various oxidizable organic compounds by introducing current by means of an anode into an electrolyte of a cell containing the oxidizable organic compound. The anode comprises an electrocatalyst material which has at least one compound of the formula:

$$A_2 \left[ B_{2-x} B'_x \right] O_{7-y}.$$

wherein A is selected from any of the pyrochlore structure metal cations of: Pb, Bi and Tl, wherein B' is selected from any of the pyrochlore structure metal cations of Pb, Bi Tl and Sn; wherein B is a pyrochlore structure metal cation, at least a major portion of which is selected from at least one of: Ru, Rh, Ir and Os, wherein y is equal to or greater than zero and equal to or less than one; and wherein x is equal to or less than one and equal to or greater than zero.

EP 0 046 054 A1

Croydon Printing Company Ltd

"A method of electrocatalytic oxidation of organic compounds"

The invention pertains to a method of reacting organic compounds electrocatalytically, and more particularly to a method of electrolytically oxidizing compounds such as alcohols, olefins, and carbonyl compounds, to carboxylates or ketones.

The following patents relate to electrocatalyst materials and their fabrication. These patents are included herein to serve as a background for the present invention. U.S. Patent Nos. 4,203,871; 4,163,706; 4,146,458; 4,129,525; and 4,124,539.

Heretofore it has been taught that pyrochlore structure electrocatalyst materials of the general formula:

$$A_2 \left[B_{2-x}B'_x\right] O_{7-y}$$

wherein A is selected from any of the pyrochlore structure metal cations of: Pb, Bi and Tl, wherein B' is selected from any of the pyrochlore structure metal cations of Pb, Bi, Tl and Sn; wherein B is a pyrochlore structure metal cation at least a major portion of which is at least one of: Ru, Rh, Ir and Os, wherein y is equal to or greater than zero and equal to or less than one; and wherein x is equal to or less than one and equal to or greater than zero, may be used in the evolution or reduction of oxygen in alkaline solutions. Efforts to explore still other uses for these novel electrocatalyst materials were undertaken when it was noted that they may have ability to change their oxide content or stoichiometry, as a function of potential in alkaline solutions. It was suggested that this behavior might be useful to initiate oxidative reactions of various oxidizable organic compounds.

Although it may be argued that the prior art has suggested using pyrochlore electrocatalyst materials for the oxidation of organic substances (see German Application No. 2150039, and U.K. Patent Specification No. 1,415,684), the electrocatalyst materials of this invention have obtained unique end products through an unusual "reaction selectivity", or "partial oxidation". To better understand what we mean by "reaction selectivity" and "partial oxidation" in terms of this invention, comparison will be made with well known oxygen producing electrocatalyst materials such as platinum. Platinum has been observed to oxidize

certain organic compounds to completion and form only carbonates as a reaction end product. This is not a useful result in the context of this invention.

By comparison, the electrocatalyst materials of this invention will catalyze certain organic substances to useful end products such as: carboxylates, carboxylic acids, ketones, etc., in a "partial oxidative" process i.e., a reaction which goes to completion without fully oxidizing the organic reactants.

The new and unexpected reaction products are often observed to be the result of unique reaction pathways or cleavages which as a group of reactions have never before been achieved by electrocatalysis to the best of the inventors' knowledge. It is believed that these uniquely "selective" results are substantially due to the particular and unusual catalytic characteristics of these pyrochlores. The high level of catalytic activity attainable with these materials can be attributed to the high surface area with which they can be fabricated, as taught in the aforementioned U.S. Patent No. 4,129,525.

Accordingly this invention relates to a method of electrocatalytically reacting an oxidizable organic compound, comprising the step of introducing current by means of an anode into an electrolyte of a cell containing said organic compound. The anode comprises the electrocatalyst material of this invention which has at least one compound of the formula:

$$A_2 \left[B_{2-x}B'_x\right] O_{7-y}$$

wherein A is selected from any of the pyrochlore structure metal cations of: Pb, Bi and Tl, wherein B' is selected from any of the pyrochlore structure metal cations of Pb, Bi, Tl and Sn; wherein B is a pyrochlore structure metal cation at least a major portion of which is selected from at least one of: Ru, Rh, Ir and Os, wherein y is equal

to or greater than zero and equal to or less than one; and wherein x is equal to or less than one and equal to or greater than zero.

The introduction of current into the electrolyte of the cell is generally made in the voltage range of between 0.3 to 5.0 volts, and more preferably between 0.5 and 1.25 volts relative to a reversible hydrogen electrode in the same electrolyte.

The above method is useful for electrocatalytically generating carboxylates from the organic compounds; primary alcohols, olefins, glycols, keto alcohols, diketones, keto acids and hydroxyacids.

The above method has been found to uniquely cleave ketones and secondary alcohols containing at least one alpha hydrogen, where an alkaline electrolyte containing said ketone or secondary alcohol is utilized. That is to say, the carbon-carbon bond next to the oxygen is broken and an acid group is formed on the new terminal carbons.

The above method has also been found to uniquely oxidize secondary alcohols to ketones in a pH range from 2 to 10. For these reactions a bismuth ruthenate or iridate has been found preferable, i.e. A and B' is Bi and B is Ru or Ir.

When olefins are to be cleaved to carboxylic acid, it has been found preferable to use a lead or bismuth ruthenate catalyst, i.e. A and B' are Pb or Bi; and B is Ru in the above formula.

For secondary alcohols and ketone cleavages, a lead or bismuth ruthenate catalyst has been found preferble, i.e., A and B' are Pb or Bi and B is Ru.

Other preferred catalyst materials feature lead and bismuth iridates.

A borate-containing electrolyte $(B_4O_7^{=})$ with a pH of approximately 9 is preferably used when secondary alcohols are reacted to provide ketones.

In general, the electrolyte may be either aqueous, non-aqueous, or a miscible mixture of aqueous and non-aqueous depending on the reactants or the reaction to be achieved. It is an object of this invention to provide a method of electrocatalytically reacting oxidizable organic compounds with the use of noble metal pyrochlores.

It is another object of the invention to use pyrochlore oxides containing noble metals as catalysts for the electrooxidation of secondary alcohols to ketones in acid or weak alkali electrolytes and the oxidative cleavage of secondary alcohols, ketones and olefinic compounds to carboxylates in strong alkali electrolytes.

These and other objects of this invention will become more apparent and will be better understood with reference to the following detailed description considered in conjunction with the accompanying drawings in which:

Figure 1 is a graphical representation of the ability of the pyrochlore crystal structure to undergo oxidative changes as a function of potential in alkaline solution;

Figure 2 is a graph illustrating the oxidative capability of a lead ruthenate catalyst; and

Figure 3 is a graph depicting the oxidation rate for various electrolyte buffers with respect to temperature.

The invention features a method of electrocatalytically reacting organic compounds. Current is introduced into a cell having an electrolyte containing the organic compound which is to be reacted. The anode of the cell contains the oxide of a noble metal pyrochlore as defined hereinbefore. The cell also comprises a cathode and means to maintain a potential difference between the cathode and anode. Typically, a reference electrode is also used in order to control the potential of the anode at some precise level relative to the

reference. It is advisable, although not absolutely necessary, to provide a separator between the cathode and anode so that hydrogen evolved from the counter electrode may be vented from the cell and, furthermore, so that the oxidized organic products produced at the anode may not come in contact with the cathode and be reduced. The anode is typically immersed in the electrolyte. The organic reactant may be introduced as a liquid or solid which may be solubilized in the electrolyte, or as a liquid which may remain immiscible and is brought into contact with the anode by thorough stirring of the immiscible components. Where the organic is an insoluble solid, it may be reacted as a slurry of fine particles. If the reactant is introduced as a gas, it may be bubbled into the electrolyte or it may be provided to the cell by means of an interface maintaining anode, which consists of a porous, wetproofed electrode that maintains a liquid electrolyte phase on one side and a gaseous reactant phase on the other, simultaneously providing an interface in its interior where electrolyte, gas and catalyst can coexist at a common interface.

Referring to Figure 1, it is seen that an anode having an oxide of a pyrochlore crystal structure (lead ruthenate) has the ability to undergo oxidative changes as a function of potential in alkaline solutions. This strongly suggests that such materials can electrocatalyze the oxidation of certain substances.

Figure 2 further illustrates the oxidative capability of a lead ruthenate anode using propylene in 0.5N KOH. An electrode containing about 300 mg of high surface area, nonstoichiometric lead ruthenate bonded to an inert gold screen current collector, was potentiostatted under nitrogen at +200 mv vs. saturated calomel (1.22 volts vs. reversible hydrogen in the same electrolyte). This raised its potential to a high level close to the theoretical potential for the reversible oxygen electrode:

$$[1]\quad O_2 + 2H_2O + 4e \rightleftharpoons 4OH^-$$

The cell was "charged" at this potential until all current due to oxide ion incorporation into the lattice decayed to zero:

$$[2]\quad Pb_2Ru_2O_{7-e} + 2\delta OH^- \rightleftharpoons Pb_2Ru_2O_{7-e+\delta} + \delta H_2O + 2\delta e$$

At this potential the oxygen evolution reaction rate and, therefore, the background current were essentially zero. The electrode was disconnected from the potentiostat and its potential remained unchanged for about an hour.

At time zero, propylene was admitted to the reaction vessel merely by bubbling it through the electrolyte. The potential of the electrode immediately began to drop, leveling off at a lower value (Figure 2). This means that reaction [2] was reversed because the propylene was oxidized.

In borate buffer at pH 9, the oxidation of propylene also occurred, and the selectivity to acetate and $CO_2$, based on the amount of carbonate isolated, was close to 100%. Runs in other buffers showed a tendency for the oxidative rates to decrease with decreasing pH as shown in Figure 3.

In order to confirm the reactivity and selectivity of the oxidation of isolated double bonds on lead ruthenate with more soluble reactants, two unsaturated carboxylic acids containing a double bond were oxidized which were far removed from the solubilizing carboxylate group. Omega undecylenic acid (11 carbon atoms, double bond next to last carbon) was no more active than propylene at 50°C but showed increased activity at 75°C.

Oxidations were carried out in aqueous solutions from pH 4.7 to strongly alkaline using a submerged electrode containing the catalyst on an inert gold screen bonded with finely divided polytetrafluoro-ethylene. The reactants were either dissolved in the electrolyte or sparged through it if gaseous.

Table I below summarizes a series of electro-oxidations on a high surface area lead ruthenate catalyst.

An olefin, propylene, was cleaved to a carboxylic acid and carbonate with high selectivity (Runs 1 and 2). No electrocatalysts other than the claimed noble metal pyrochlores are known to do this so selectively. Primary alcohols were oxidized to the corresponding carboxylates very selectively, without formation of $CO_2$ due to the further oxidation of the product (Runs 3 and 4).

A secondary alcohol, (secondary butanol) was oxidatively cleaved in alkali to two moles of acetic acid. The expected intermediate, methyl ethyl ketone was also cleaved to the same product as was 2,3 butanediol (Runs 5,6,7 and 8). In weaker alkali however, secondary butanol consumed only 2 electrons/molecule and formed methyl ethyl ketone--no acetate was detected (Run 9).

TABLE I

| Run | Catalyst | Medium | Temp. °C | Reactant | Products | Selectivity % |
|---|---|---|---|---|---|---|
| 1 | $Pb_2[Ru_{1.66}Pb_{.34}]O_{6.5}$ | 1M KOH | 50 | $C_3H_6$ | $CH_3COO^-+CO_3^=$ | 100 |
| 2 | $Pb_2[Ru_{1.67}Pb_{.33}]O_{6.5}$ | $Na_2B_4O_7$ | 50 | $C_3H_6$ | $(CH_3COO^-)+CO_3^=$ | ~100 |
| 3 | " | 1M KOH | 50 | $C_2H_5OH$ | $CH_3COO^-$ | 94 |
| 4 | $Pb_2[Ru_{1.68}Pb_{.32}]O_{6.5}$ | 1M KOH | 50 | $C_3H_7OH$ | $C_2H_5COO^-$ | ~100 |
| 5 | $Pb_2[Ru_{1.66}Pb_{.34}]O_{6.5}$ | 1M KOH | 25 | $\eta C_4H_9OH$-2 | $2CH_3COO^-$ | 67 |
| 6 | " | 1M KOH | 50 | $\eta C_2H_5CO-CH_3$ | $2CH_3COO^-$ | 81 |
| 7 | " | 1M KOH | 50 | $\eta C_4H_9OH$-2 | $2CH_3COO^-$ | 76 |
| 8 | " | 2M KOH | 25 | $CH_3(CHOH)_2CH_3$ | $2CH_3COO^-$ | 84 |
| 9 | " | .125M $Na_2B_4O_7$ | 50 | $C_2H_5CHOH-CH_3$ | $C_2H_5COCH_3$ | 89 (55*) |
| 10 | " | 1.5M KOH | 50 | $CH_2-(CH_2)_4-CO$ | $(-C_2H_4COO^-)_2$ | 92 |
| 11 | " | 1.5M KOH | 50 | $CH_2(CH_2)_4CHOH$ | $(-C_2H_4COO^-)_2$ | 89 |
| 12 | " | 1.5M KOH | 50 | $CH_2=CH-(CH_2)_8COO^-$ | $(-C_4H_8COO^-)_2+CO_3^=$ | - |
| 13 | " | 2M KOH | 75 | 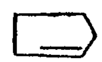$-CH_2-COO^-$ | 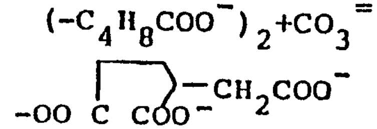 | 85 |

* Yield low due to evaporation losses.

Similar oxygenate cleavages were demonstrated with cyclohexanol and cyclohexanone, which formed adipic acid (after acidification) (Runs 10 and 11).

Further olefinic cleavages are demonstrated with undecylenic acid which was oxidized to sebacic (+ some azelaic) and 2 cyclopentene-1-acetic acid which underwent oxidation to a tricarboxylic acid (Runs 12 and 13).

In Table II below are shown oxidations carried out on bismuth ruthenate. Run 14 illustrates a ketone cleavage while runs 15 and 16 show an olefin cleavage: maleate cleaving to 2 moles of oxalate at pH 4.7 and in strong alkali. Run 17 shows the inactivity of the product oxalate to further oxidation. Sintered nickel, a known catalyst capable of oxidizing primary alcohols, proved to be inert to maleic acid, secondary butanol, and methyl ethyl ketone under the same conditions where lead and bismuth ruthenates were active (Runs 18, 19, 20 and 21). Ruthenium dioxide was also active for the oxidation of methyl ethyl ketone (Run 22) but is not useful because of its tendency to dissolve in alkali as ruthenate ion. Even platinum black (not shown in the tables) does not have the capability of these catalysts. With ethanol in alkali, Pt formed considerable carbonate rather than acetate and deactivated before the substrate was half consumed. With either maleic acid or secondary butanol in alkali, Pt was entirely inactive.

TABLE II

ELECTROORGANIC OXIDATIONS INVOLVING DOUBLE BOND CLEAVAGE

| Run | Electrolyte | Catalyst | Potential (mV vs. RHE) | Reactant | Selectivity % |
|---|---|---|---|---|---|
| 14 | 1M KOH | $Bi_2[Ru_{1.25}Bi_{.75}]O_{7-y}$ | 1201 | MEK | 95.6 (1) |
| 15 | 2M KOH | " | 1262 | Maleic Acid | 59.2 (2) |
| 16 | HAc/NaAc pH=4.7 | $Bi_2[Ru_{1.25}Bi_{.75}]O_{7-y}$ | 1210 | Maleic Acid | Active |
| 17 | 2M KOH | " | 1222 | Oxalic Acid | Inactive |
| 18 | 3M KOH | Sintered Ni° | 1408 | Maleic Acid | Inactive |
| 19 | 3M KOH/75°C | " | 1394 | Maleic Acid | Inactive |
| 20 | 3M KOH | Sintered Ni° | 1441 | Sec Butanol | Inactive |
| 21 | 1M KOH | " | 1441 | MEK | Inactive |
| 22 | 1M KOH | $RuO_2$ | 1241 | MEK | 100 (1) |
| 23 | 1:1 1M KOH/Sulfolane | $Bi_2[Ru_{1.25}Bi_{.75}]O_{7-y}$ | 1250 | Maleic Acid | 66.7 (2) |
| 24 | 2M KOH | $Bi_2[Ru_{1.72}Bi_{.28}]O_{7-y}$ | 1216 | Acrylic Acid | 69.8 (3) |

(1) Product acetate identified by NMR. Amt. determined by titration with HCl. Selectivity based on the reaction:

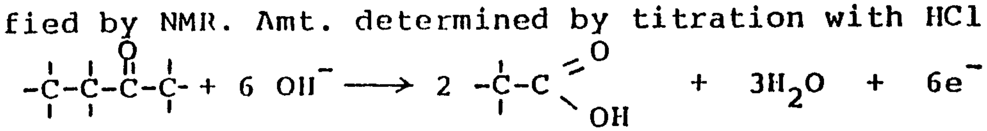

(2) Product oxalate recovered by precipitation with $Ca(NO_3)_2$. Identified by x-ray diffraction and amount determined gravimetrically. Selectivity based on the reaction:

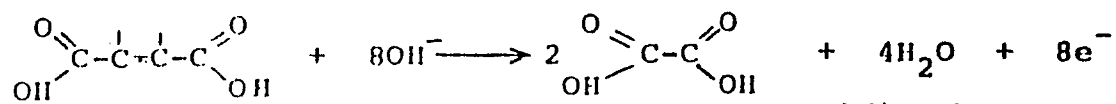

(3) Product oxalate recovered by precipitation with $Ca(NO_3)_2$. Identified by x-ray diffraction and amount determined gravimetrically. Selectivity based on the reaction:

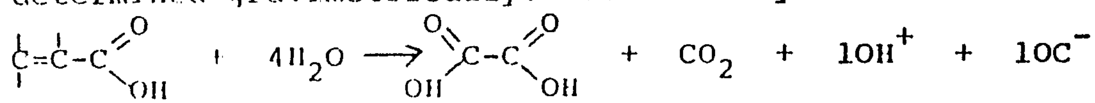

Table III below shows the results of electro-oxidations carried out on ethanol and propanol with various catalysts. It will be seen that high specific surface area is another prerequisite for the activity of the pyrochlore, and that sintered nickel, $RuO_2$ and $NiCo_2O_4$ can match their selectivity whereas other low surface area oxides cannot. A comparison of Table III with Table II shows that whereas several electrocatalysts can promote the oxidation of primary alcohols to carboxylates none can match the unique ability of the noble metal pyrochlores to cleave olefins, secondary alcohols and ketones.

TABLE III

ELECTROORGANIC OXIDATION OF PRIMARY ALCOHOLS

| Run | Electrolyte | Catalyst | S.A. | Potential (mV vs. RHE) | Reactant | Selectivity %[(1)] |
|---|---|---|---|---|---|---|
| 24 | 1M KOH | $Pb_2[Ru_{1.61}Pb_{.39}]O_{7-y}$ | 145 | 1290 | $C_2H_5OH$ | 100 |
| 25 | 1M KOH | $Pb_2Ru_2O_{6.5}$ | 3.3 | 1290 | $C_2H_5OH$ | 98.5 |
| 26 | 1M KOH | $Bi_2Ru_2O_7$ | 0.2 | 1440 | $C_2H_5OH$ | Inactive |
| 27 | 1M KOH | $Bi_2[Ru_{1.46}Bi_{.54}]O_{7-y}$ | 157 | 1220 | $C_2H_5OH$ | 100 |
| 28 | 1M KOH | " | " | 1221 | $C_3H_7OH$ | 99.4 |
| 29 | HAc/NaAc pH=4.7 | " | " | 1210 | $C_3H_7OH$ | Active for propionate-Selectivity not determined |
| 30 | 1M KOH | $PbO_2$ | 0.3 | 1440 | $C_2H_5OH$ | Inactive |
| 31 | 1M KOH | $RuO_2$ | 14 | 1240 | $C_2H_5OH$ | 100 |
| 32 | 1M KOH | $RuO_x$ | 145 | 1240 | $C_2H_5OH$ | Unstable |
| 33 | 1M KOH | $BaPbO_3$ | 0.8 | 1440 | $C_2H_5OH$ | Inactive |
| 34 | 1M KOH | Sintered Ni° | | 1440 | $C_2H_5OH$ | 98.2 |
| 35 | 1M KOH | $NiCo_2O_4$ | 64 | 1240 | $C_2H_5OH$ | 97.3 |

All oxidations carried out at 50°C.

(1) Product in each case was the corresponding carboxylic acid. Amt. determined by titration with HCl. Identified by NMR. Selectivity calculated on the basis of a 4 electron oxidation of the alcohol to the carboxylic acid.

In addition to acidic and basic aqueous electrolytes, these pyrochlores can be used as electro-oxidation catalysts in electrolytes containing a significant organic component. This is important to aid the solubility of organic reactants. Maleic acid is found to be very reactive in a 1:1 1m KOH/sulfolane electrolyte as shown in Run 23 - Table II.

The use of "expanded" noble metal pyrochlores which have varying amounts of lead and bismuth replacing the noble metal are particularly well suited for the electro-oxidation of organics. This is because they can be prepared with the highest surface area of all pyrochlores. Other advantages of this class of materials are their high electrical conductivity, range of oxygen nonstoichiometry and lower noble metal content per unit activity.

CLAIMS

1. A method of electrocatalytically reacting an oxidizable organic compound comprising the step of introducing current by means of an anode into an electrolyte of a cell containing said oxidizable organic compound, said anode comprising an electrocatalyst material having at least one compound of the formula:

$$A_2 \left[ B_{2-x} B'_x \right] O_{7-y}$$

wherein A is selected from any of the pyrochlore structure metal cations of: Pb, Bi and Tl, wherein B' is selected from any of the pyrochlore structure metal cations of Pb, Bi, Tl and Sn; wherein B is a pyrochlore structure metal cation, at least a major portion of which is selected from at least one of: Ru, Rh, Ir and Os, wherein y is equal to or greater than zero and equal to or less than one; and wherein x is equal to or less than one and equal to or greater than zero.

2. A method according to claim 1, wherein A and B' are Pb.

3. A method according to claim 1, wherein A and B' are Bi.

4. A method according to either of claims 2 and 3 wherein B is Ru.

5. A method according to either of claims 2 and 3 wherein B is Ir.

6. A method according to any one of the preceding claims wherein said electrolyte is an aqueous solution.

7. A method according to any one of claims 1 to 5 wherein said electrolyte is a non-aqueous solution.

8. A method according to any one of claims 1 to 5 wherein said electrolyte is a mixture of aqueous and non-aqueous solutions.

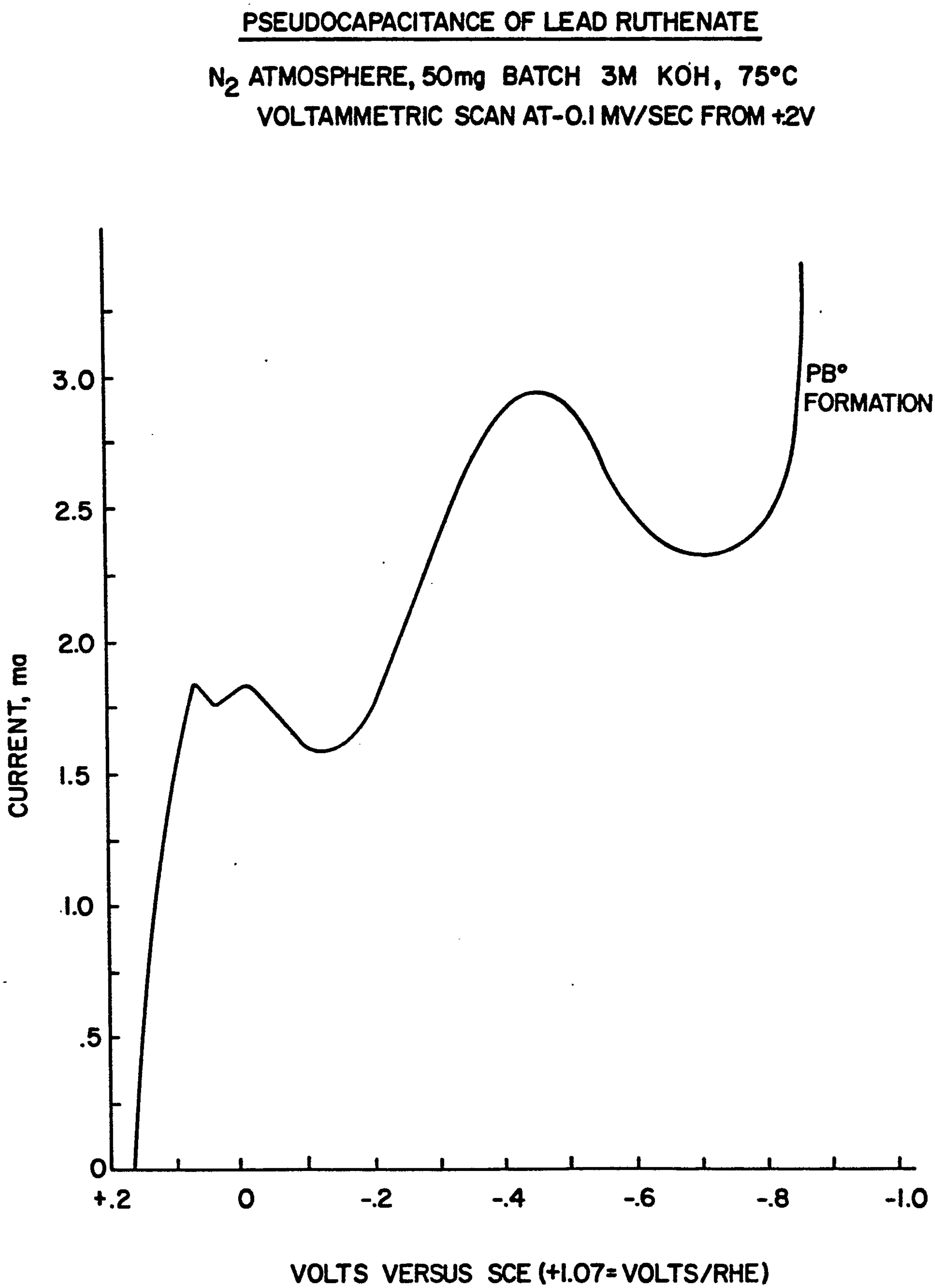

PSEUDOCAPACITANCE OF LEAD RUTHENATE
$N_2$ ATMOSPHERE, 50mg BATCH 3M KOH, 75°C
VOLTAMMETRIC SCAN AT-0.1 MV/SEC FROM +.2V
CURRENT, ma
3.0
2.5
2.0
1.5
1.0
.5
0
+.2
0
-.2
-.4
-.6
-.8
-1.0
PB°
FORMATION
VOLTS VERSUS SCE (+1.07=VOLTS/RHE)


FIG.1

0046054

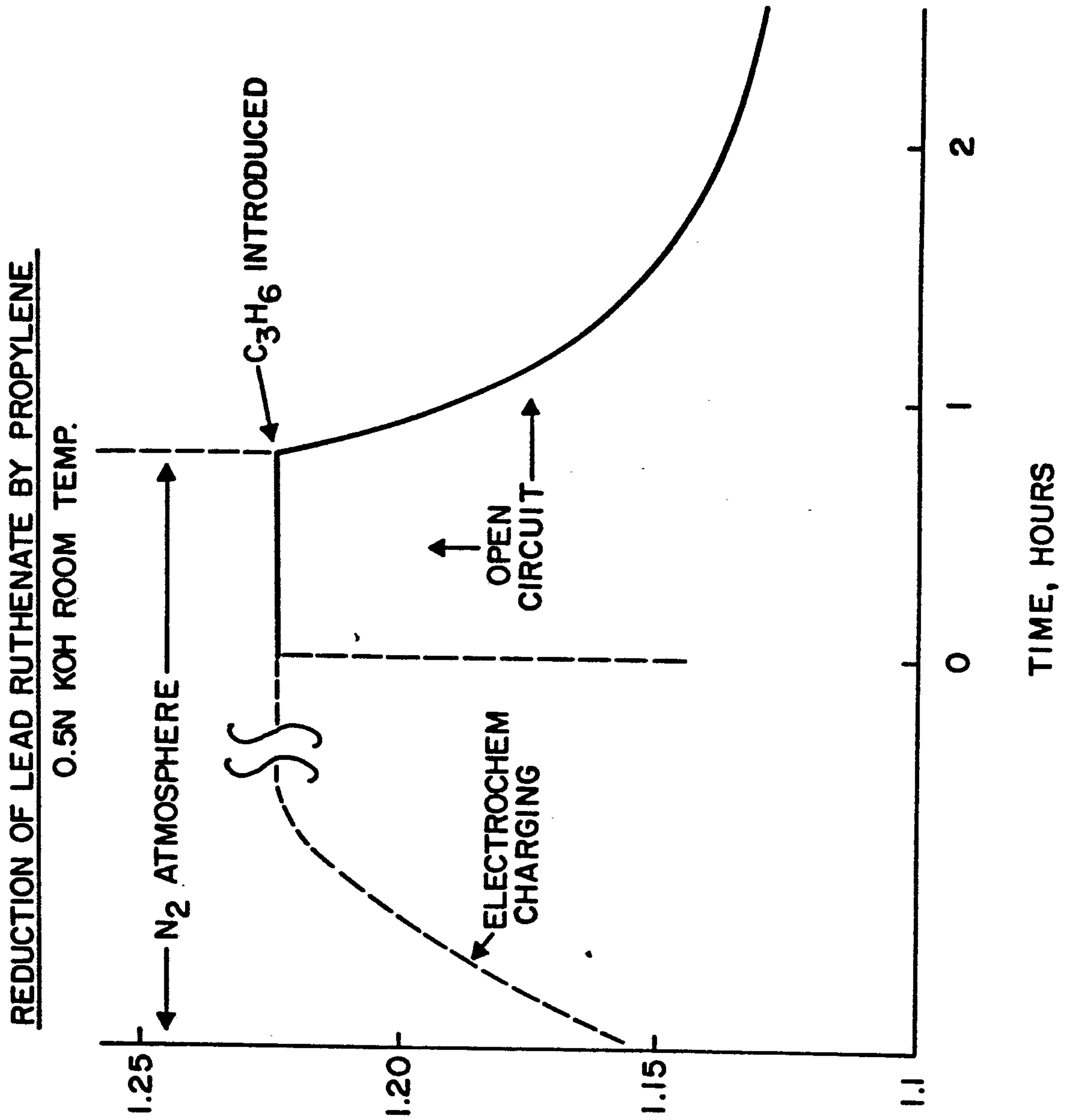


FIG. 2

RATES OF OXIDATION OF VARIOUS ORGANICS ON LEAD RUTHENATE

V= 1.2 VOLTS VS. RHE

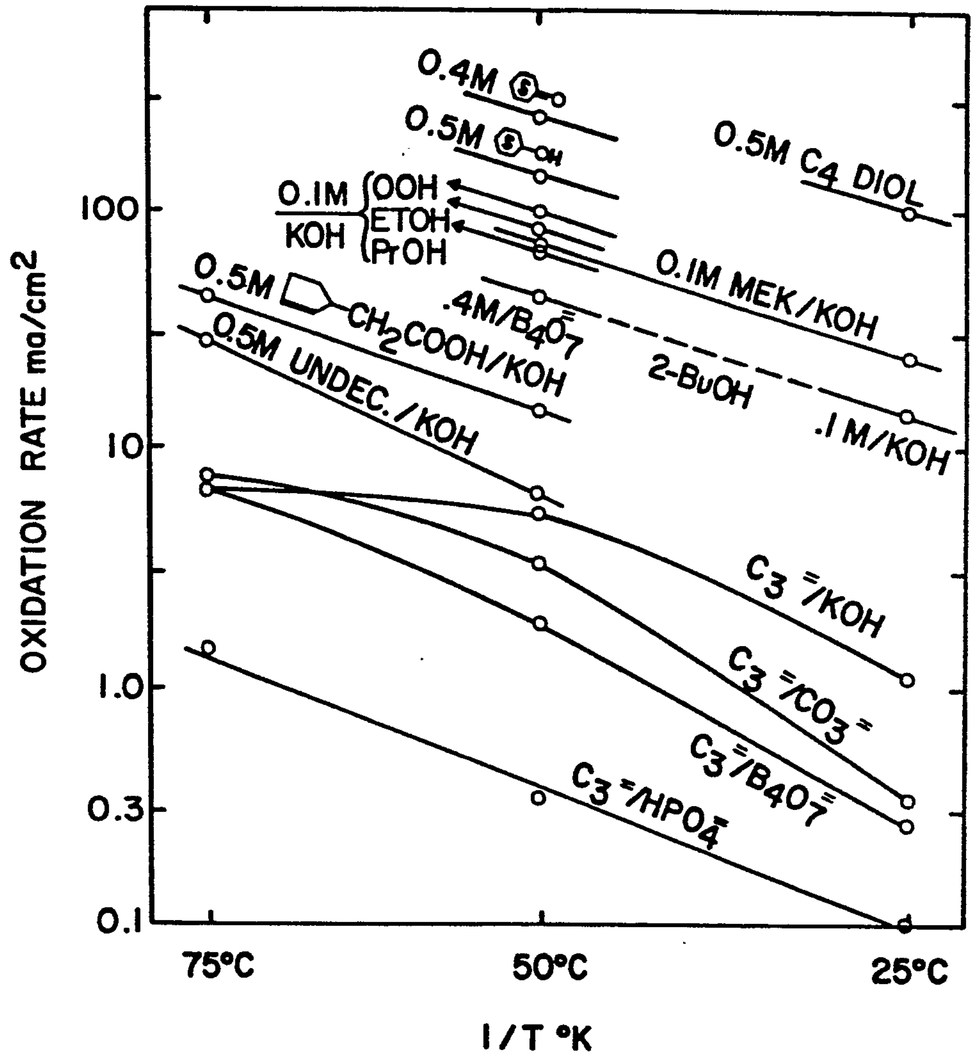


FIG. 3

0046054

European Patent Office

**EUROPEAN SEARCH REPORT**

Application number

EP 81303574.8

| Category | Citation of document with indication, where appropriate, of relevant passages | Relevant to claim | CLASSIFICATION OF THE APPLICATION (Int. Cl.³) |
|---|---|---|---|
| P | EP - A1 - 0 014 596 (DIAMOND SHAMROCK CORPORATION(20-08-1980)<br>* Claim 1 * | 1 | C 25 B 3/02<br>C 25 B 11/06<br>C 07 C 53/08<br>C 07 C 55/02<br>C 07 C 55/22<br>C 07 C 49/10 |
| A | GB - A - 1 195 871 (CHEMNOR AG)<br>* Claims 1-4; page 2, lines 16-40 * | 1 | |
| A | US - A - 4 052 271 (HENRI BERNARD BEER)<br>* Claim 1 * | 1 | |

TECHNICAL FIELDS SEARCHED (Int. Cl.³)

C 25 B

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| VIENNA | 07-10-1981 | HEIN |